# EUROPEAN PATENT SPECIFICATION

(11) **EP 3 137 893 B1**
(45) Date of publication and mention of the grant of the patent: **15.11.2023**
(21) Application number: 15720675.6
(22) Date of filing: 28.04.2015
(51) Int. Cl.: G01N 21/85, G01N 21/31, G01N 33/12, G01N 21/49

(54) **MEASUREMENT OF PROPERTIES OF AN ORGANIC MATERIAL**
MESSUNG VON EIGENSCHAFTEN EINES ORGANISCHEN MATERIALS
MESURE DES PROPRIÉTÉS D'UN MATÉRIAU ORGANIQUE

(30) Priority: 28.04.2014 NO 20140543
(43) Date of publication of application: 08.03.2017
(73) Proprietor: Sintef TTO AS, 7465 Trondheim (NO)
(72) Inventor: TSCHUDI, Jon, 0654 Oslo (NO); O'FARRELL, Marion, 0373 Oslo (NO)
(74) Representative: Bryn Aarflot AS
(86) International application number: PCT/EP2015/059162
(87) International publication number: WO 2015/165885

(56) References cited:
- EP-A1- 0 374 844
- WO-A1-2004/106899
- WO-A1-2007/000165

## Description

This invention relates to a system for measuring the properties of an organic material for example being subject to an external influence altering certain optical properties of the material. The invention is especially related to organic materials like meat, including fish and white meat, being heated.

Monitoring meat or similar during cooking has traditionally been performed by measuring the temperature of the meat and comparing the temperature with known properties of the meat, e.g. stating that the meat is considered to be medium rare at 65°C. This is, however, a slow measurement and thus the processing may be stopped too late. Also it is not sufficiently reliable as it depends on the position of the sensor and the kind of meat being prepared, and often the meat is cut in order to inspect the colour before it is decided that it is at the desired endpoint.

WO2004/106899 describes a method for measuring the colour of the meat by transmitting light into the meat and measuring the specter of the light reflected by the material. Although the solution is close to the practical, manual inspection it has some disadvantages as the measurement is very local close to the sensor and the spectrum is difficult to measure as the reflection will vary to a large degree depending on meat type as well as age and general conditions. Similar solutions are also discussed in EP0416658, EP0402877, US5239180, US6118542 and DE10109246, while US6563580 measures the direct transmission through a short path length through the meat.

WO 2007/000165 A1 also describes a measuring instrument to be inserted into a material measuring the absorption through the material between two parts transmitting the optical interrogation signal directly between them. WO 2007/000165 A1 discloses the features of the preamble of appended claim 1. A similar solution is described in EP 0 232 802 A1. EP 0 374 844 A1 describes a multipath scattering measurement system.

Thus, it is an object of the present invention to provide a measuring system providing a reliable optical solution for monitoring and inspecting materials like meat, fish etc. This is obtained as described in the accompanying claims.

Thus due to the increased speed of the measurement in comparison to traditional temperature measurements it is possible to measure the desired properties continuously as the probe is inserted. Furthermore, it is possible to include depth sensing for profiling the measured properties along the depth.

Thus the present invention both takes advantage of measuring only the parts of the spectrum relevant for the chemical reactions or scattering properties relating to another processes such as denaturisation of proteins in the organic material as well as investigating a volume of the material by monitoring light having passed through it a predetermined length.

The invention will be described more in detail with reference to the accompanying drawings, illustrating the invention by way of examples.
- Figure 1a,b: illustrates a probe according to the invention
- Figure 2a-f: illustrates different embodiments of the probe used for the measurements according to the invention.
- Figure 3: illustrates the measuring unit according to one embodiment of the invention.
- Figure 4a,b: illustrate two possible measuring sequences for selectively measuring the spectrum ranges..
- Figure 5a,b: illustrates an example of chemical reaction and the changes in the spectrum related to one known reaction in heated meat.
- Figure 6a,b: illustrates differences between spectra in beef and chicken meat.
- Figure 7: is a flow chart illustrating a preferred use of the present invention.

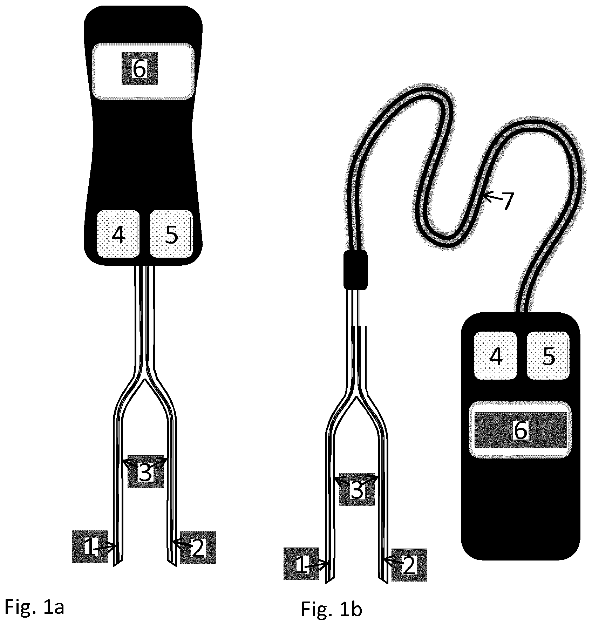

As is illustrated in figure 1a and b the present invention primarily relates to a system or unit for measuring the properties of an organic material using a fork, ferrule or similar 1,2,3 to be introduced into the material. The ferrules may have thickness making it easy to introduce into the material, 2-5mm for practical reasons, but possibly down to 1mm. The required thickness makes it preferable to use light guides such as optical fibres in the ferrules conducting the light to and from an electronics unit 6.

In figure 1a the unit is constituted by a fork having two ferrules 3 each including light guides 1,2 for conducting light into the organic material and from it into a handle comprising light source unit 4, light detector unit 5 and electronic circuitry 6 such as analyzing unit, display, etc. By positioning the electronics outside the ferrules and in the electronics unit 6 is to avoid exposing the circuitry to the temperate variations that may be applied on the organic material.

As will be discussed below both the light source unit and the detector unit may be of different types, the important aspect being that it is transmitted and received at the wavelengths necessary to provide relevant measurements. Thus the light source unit 4 may be a single light source emitting light with a large range of wavelengths and the corresponding receiver unit may include a number of detectors being filtered or otherwise adapted to receive light within the specified wavelengths. Compatible pairs of sources and detectors may be used or a multiplexing scheme as discussed below. If some properties of the organic material are known, e.g. being red meat, the detection may be limited to a subset of the wavelength ranges selected for measuring of the desired spectral features so as to make the detection easier.

In figure 1b a flexible light guide bundle 7 is used between the fork 3 and the detector and processing unit, e.g. to the light source, detector and processing unit 4,5,6. The unit may then also be implemented as a part of existing equipement such as an oven.

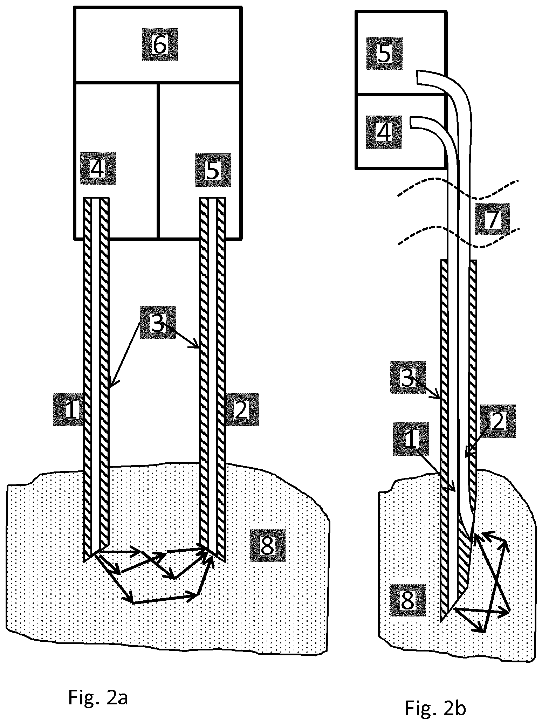

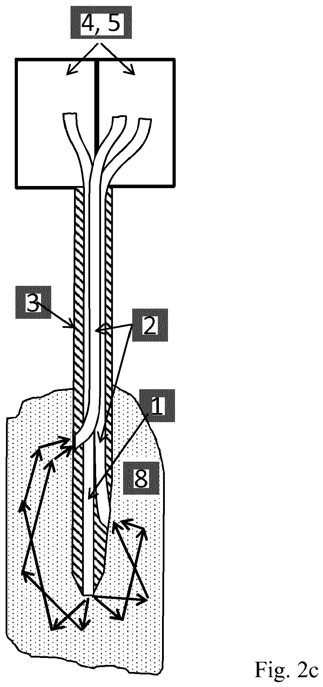

In figure 2a,b,c three embodiments of the invention are illustrated showing different light propagations through the material. In figure 2a an implementation with separate ferrules for illumination and detection, as in figure 1a,b, showing how the light propagates through the material 8 from the first ferrule 1 coupled to the light source 4 to the second ferrule 2 coupled to the detectors. The tip can be slanted to enable easier insertion. As can be seen from the drawing the light guide ends are not aimed directly at each other, so it is not only light transmitted directly through the material that is measured but also light scattered in the material. (this is often described as 'interactance' or "transflection" in literature). The direction of the light guide ends may be chosen depending on the situation but not be aimed directly at each other (this is often described as transmission in literature).

The quality of the measurement will depend on the distance between the illumination- and detection fiber. A long distance will increase the volume that is measured, making the result less sensitive to random variations in the object. Weak features in the spectrum will also become more prominent and easier to measure. The intensity of the light (signal strength) will however be strongly reduced when the distance increases, in particular in wavelength regions where the scattering and/or absorption is large. The optimal distance can therefore depend on selected wavelength ranges and properties of the measured material, and the selected distance should be a compromise giving reasonable performance for the desired materials and wavelength ranges. Tests have shown that distance above 15mm gives very low signal, and if the distance is below 1mm the result will be closer to a reflection measurement with less prominent features. For many measurement situations, a distance of 3-8mm will be preferable, but dependent on the material and situation.

In figure 2b only one ferrule is used where the light enters into the material at the ferrule end and scattered light is received by a second light guide 2 on the side of the ferrule. In addition, figure 2b illustrates the light guide bundle 7 as also mentioned in relation to figure 1b.

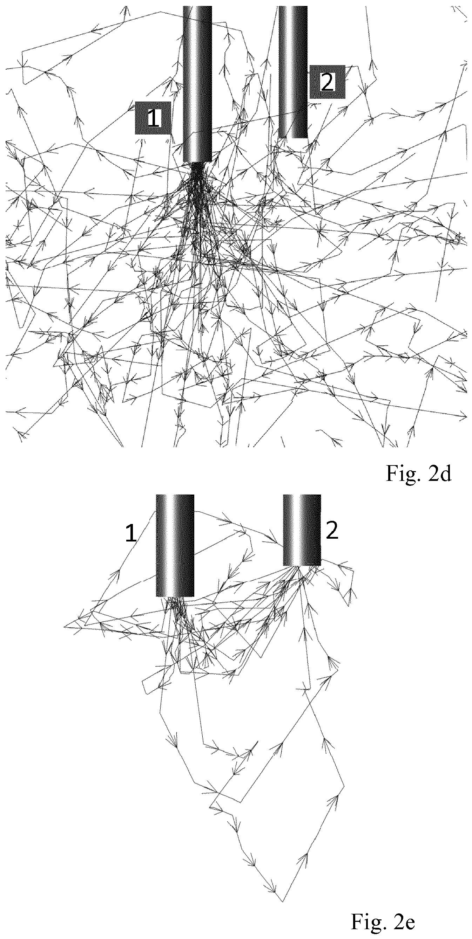

In figure 2c an additional receiving fibre makes it possible to measure at two path lengths. A possible implementation is to connect the additional light receiving fiber 2 to a separate detector unit such that both paths can be measured simultaneously, i.e. two detector unit/one source configuration. This can also be done vice versa, i.e. one detector unit/two illumination sources configuration Since the optimal distance is dependent on the wavelength range and material to be measured, as discussed above, an instrument with two distances will be more flexible and it will be possible to extract more accurate information about the measured material, e.g. based on local differences in the material or if the spectrum depends on the scattering distance (e.g. if the material is relatively transparent). Using for example one light source point and two detector points being positioned at different distances along the ferrule from the light source extracts more scattering information. Alternatively, using for example one light source point and two detector points being positioned at equal distances along the ferrule but on opposite sides of the source point an indication of the profile may also be measured.Fig 2d illustrates a simplified model of a highly scattering medium. The directions of the photons are randomized after a short distance. The relative orientation (angle) of the emitting and receieving means is of less importance when the distance between them is sufficiently long compared to the mean free path of the photons.

Fig 2e is essentialy similar to figure 2d. A much larger number of rays are traced, but only the rays reaching the detecting means with remaining energy more than 1% are shown, which will amount to about 1 out of 1000 rays in this example.

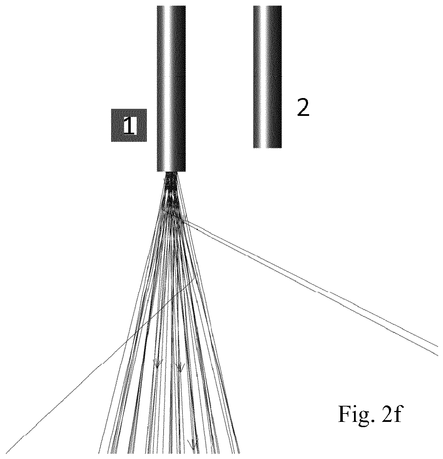

Figure 2f illustrates a situation with little or no scattering in the material, which is a situation that is not suitable for our invention.

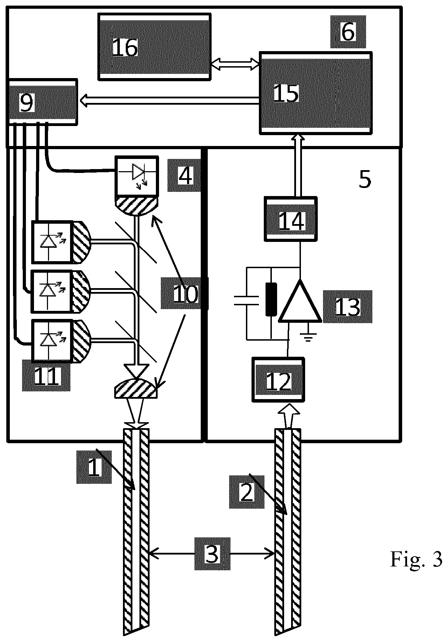

Figure 3 illustrates the preferred embodiment of the invention using by multiplexing LEDs with different wavelengths into one waveguide, and using a simple detector on the other waveguide. The light source unit 4 comprises a number of LEDs 11 being coupled into the light guide 1 through suitable optics 10. A controller 15 is coupled to the LED driver 9 in order to emit light from the LEDs in a sequence. If the LEDs have difference spectra a sequence of different wavelength ranges will be transmitted into the organic material and received through the receiving light guide 2 to a detector 12, amplifier 13 and ADC circuitry 14 in the detector unit 5. The signals are then processed by the controller 15 and the result displayed on user interface such as a display. This provides an efficient means for calibration, background correction and distinguishing the different wavelength ranges as well as will be discussed below.

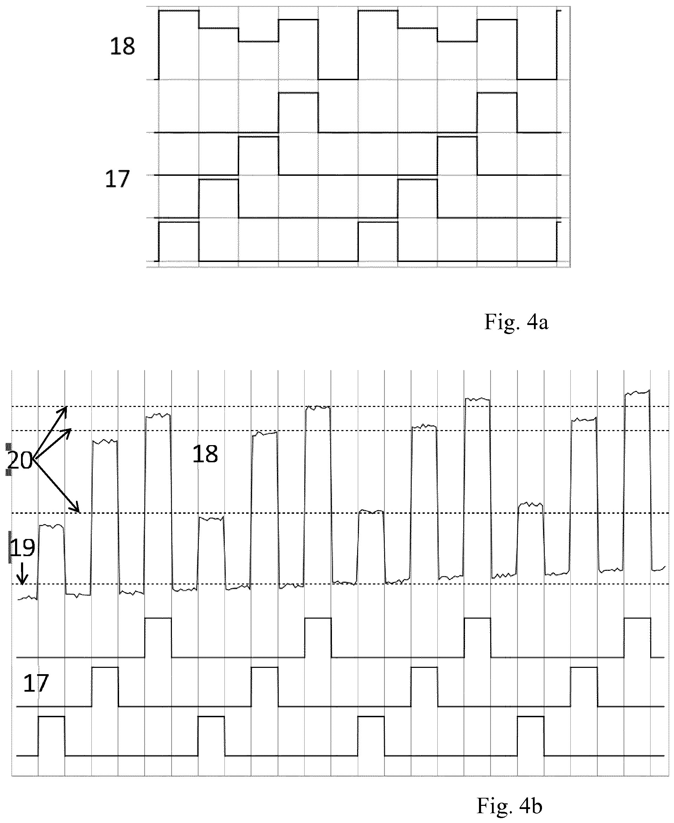

Figure 4a illustrates this possible scheme, using time-multiplexing of the LEDs, and including a period when all LEDs are off to compensate for surrounding light. The lower four traces 17 shows the signals for controlling four LEDs, the upper trace 18 shows the resulting detector signal. Some or all of the LEDs can be replaced with suitable laser diodes, for example in cases where narrower bandwidths are required or if more efficient fibre-coupling is required.

The analysis of the data in the example shown in figure 4a may include averaging, e.g. for 10 cycles for SNR. The LEDs relate to wavelength selection, the typical cycle time may be in the range of 10ms, with a total measurement of 100ms. Calibration periodes may also be included in the sequence, measuring background levels and white balance.

Fig 4b illustrates an example of a part of a sequence with 3 LEDs, showing four cycles. These cycles will be repeated for the complete measurement. The traces 17 shows the controlling signal for each LED. 18 shows the resulting detector signal, including noise and varying background. The background adds to all the measurements and includes contributions from ambient light, dark current in the detector, offset in the amplifier and low frequency electrical noise. At least one dark period with all LEDs off should be included for each cycle such that this background can be calculated and then subtracted from the LED measurements. In figure 4b, a dark period is included between each LED, giving a total of 3 dark measurements for each cycle. The dotted line 19 is calculated from these dark measurements and shows the average level from ambient light, amplifier offsets etc, while the three dotted lines 20 shows the average level when each of the 3 LEDS are turned on. The resulting measured levels are the differences between each of the 3 levels 20 and the dark level 19, averaged over the desired number of cycles. The quality of the measurement is greatly improved by measuring the background signal and calculating these differences, the most important aspect being correction of the unpredictable contribution from ambient light that penetrates the piece to be measured.

Other solutions for discriminating between the wavelength ranges may be used, e.g. using a broad band light source and filtered detectors in the detector unit. A small incandescent bulb and a miniature grating spectrometer could be a good combination for a more advanced instrument, Recent development in low-cost spectrometers could make this feasible, for example the Mini-spectrometer MS series C11708MA from Hamamatsu (http://www.hamamatsu.com/eu/en/C11708MA/index.html).

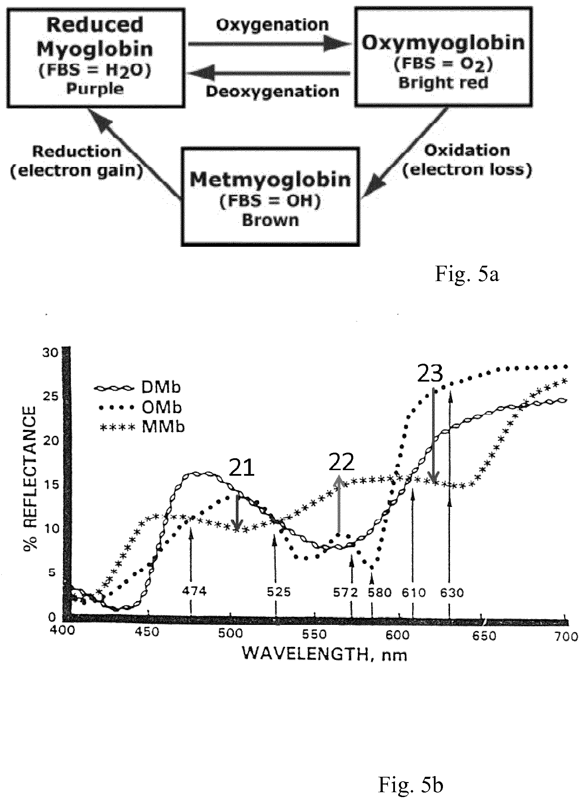

Figure 5a illustrates a flow chart for reduced myoglobin to oxymyoglobin to metmyoglobin formation, the chemical processes that cause colour in red meat. There are 3 chemical states of myoglobin: Oxymyoglobin (OMb), Deoxymyoglobin (DMb) and Metmyoglobin (MMb), depending on whether meat has been exposed to oxygen or not. The colours for these are: OMb - red, DMb- purple, MMb-brown. When beef is cooked, a hemichrome pigment is created which gives meat its brown cooked colour. This hemichrome is comprised of denatured globin and oxidized heme iron (MMb) and is a permanent state if the globin is fully denatured.). The spectra for these three states are illustrated in figure 5b illustrating the reflectance at three different wavelengths 21,22,23, and as may be seen the first reflectance 21 at about 500nm is reduced during the transition from OMb or DMb to MMb. The second reflectance 22 is increased at about 570nm from OMb or DMb to MMb, and at 620nm the third reflectance 23 decreases from DMb or OMb to MMb. The similar relative changes will apply for interactance or transmission.

This illustrates how measurements at these three wavelengths will make it possible to measure the state of the meat by recognizing the differences between how the different parts of the spectrum evolves during the processing. In use the system may signal that the meat has reached a specific state when the change at all three wavelengths each has reached a certain value, or cluster analysis or other techniques may be used for providing a measure based on the relative changes between the different measurements.

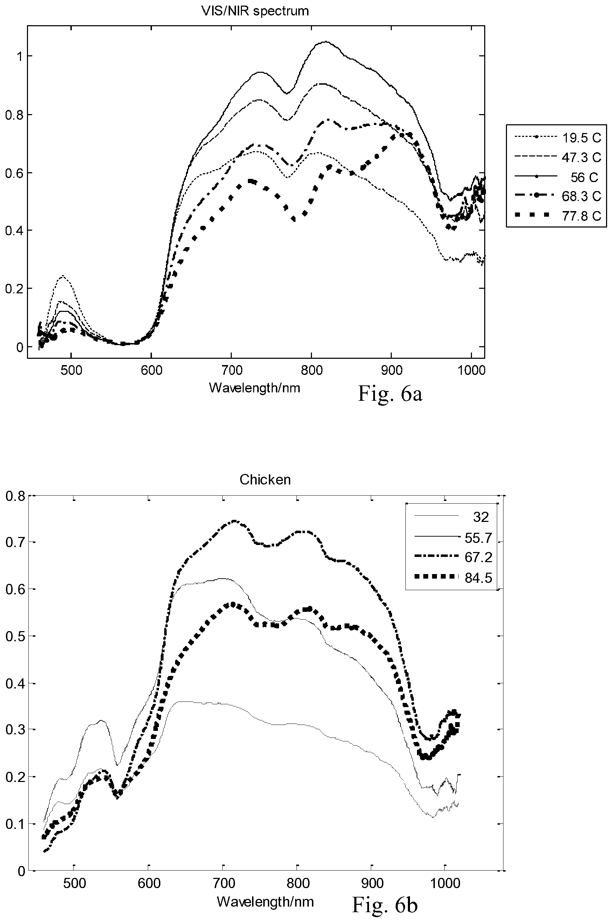

Figure 6a illustrates the spectrum at a number of different temperatures in beef. In spectral regions with relatively low absorption of light, e.g. 700-1000nm for beef, a distance of 6-10mm between the fibres can be desirable to amplify the absorption from weaker features in the spectrum. For other wavelengths, e.g. 450-650nm for beef, the light loss due to absorption and scattering is much higher, and a shorter distance of e.g. 3-5mm will give more useful information from the measurement in this wavelength range.

In figure 6b a corresponding spectrum is shown for chicken and as can be seen the differences are sufficiently large to make it possible to distinguish between the different types of meat. Thus when preparing a meal the system may automatically choose the correct mode depending on the detected type of meat, and for example suggest the preparation to the user.

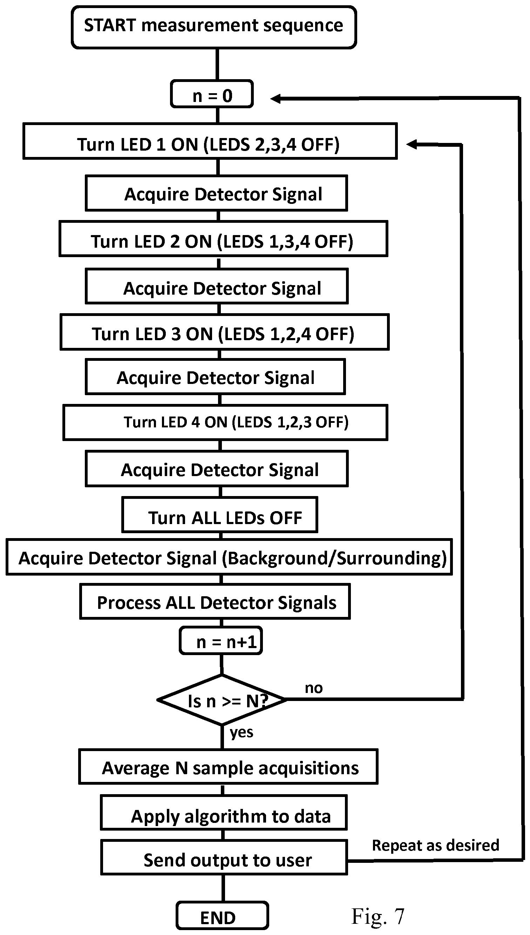

The flow chart in figure 7 shows an example of a measurement sequence where 4 LEDS are used, e.g. as illustrated in figure 4a, to illuminate and there is one background reading per cycle. N defines the number of cycles to be averaged in one measurement. One LED and thus wavelength range, is illuminated at a time in a sequence while the others are turned off, and the sequence is followed by a dark period for acquiring a detector signal relating to background, light from the surroundings etc. Additional dark periods might be inserted between the LEDs to improve the background correction, as shown in figure 4b.

Several measurements can be made at a fast rate and this creates a measurement sequence. To allow profiling at a typical insertion speed, the measurement should be calculated and displayed approximately every 100ms. This will be perceived as instant and continuous by the user. This high update-speed is much faster than existing food thermometers and allows for identification of the least-cooked point in the material if inserted all the way through the thickest part, and makes it possible to display the profile in some graphical manner. For continuous monitoring of the cooking process at a single position a much slower measurement time is sufficient.

In the end an average of the sampled measurements may be calculated, the measurements is compared with known data concerning the relevant meat types and related chemical reactions, and an output signal may provide an indication to the user, e.g. indicating the status of the material.

Thus to summarize the present invention relates to a measuring system for measuring the properties of an organic material, e.g. meat. The system comprising a light source unit, preferably constituted by at least two LEDs, emitting light within at least one chosen range of wavelengths. The light source unit may be constituted by one source emitting light within a wavelength range including all the relevant ranges to be measured, or any suitable number of sources covering the necessary ranges. The light source unit is being coupled to a light guide in a ferrule being adapted to be introduced into said material so that the light guide transmits light to at least one point within the material.

The system also comprises detector means for being adapted to receive light within said at least two wavelength ranges comprised within said emitted range of wavelengths, having passed through a chosen length in said material. The detector means thus receiving the light from at least one point within the material. Preferably the detector means is constituted by at least one light guide extending from at least one point in a ferrule chosen so as to be inside the material toward at least one light sensor for measuring light within the wavelength ranges to be measured.

The system also comprises analyzing means for repeating the measuring of the light received at the detector(s) over time in a predetermined sequence, and evaluating the condition of the material based on the measured attenuation due to the combination of scattering and absorption in the material in said at least two differently-evolving ranges of wavelengths. Thus the relative variation in the attenuation between the wavelengths may be used to evaluate the condition of the material.

The wavelength ranges are chosen based on known chemical reactions in the material during heating, so as to provide an indication of state of condition during the heating. Preferably the material is red meat or fish and the chemical reactions are related to the preparation of meat, e.g. indicating the colour of the prepared meat.

The wavelength ranges may be in the ranges being optimized for the for measuring protein denaturation during heating, including near IR, especially 900-1000nm, or possibly in the ranges of 600-650nm, 700-720nm, 750-780nm and 790-840nm.

The analysis is based on a cluster analysis based on the measured optical characteristics of the material, or other methods for analyzing and comparing a number of parameters.

The light source unit may be constituted by a light bulb or other broad spectrum source, e.g. when using a spectrometer at the receiver as discussed above, but may alternatively be constituted by at least one light emitting diode emitting light within said chosen wavelength ranges., and the at least one detector for measuring the received light within each wavelength range.

Using at least two detectors or light sources for each wavelength range it is possible to define a number of different propagation lengths through the material between the light sources and detectors by choosing the relative positions of the light source points transmitting the light into the material and the point light is received in the material and transmitted to the sensors.

While the drawings illustrate the light source and detector positioned in the handle outside the meat one embodiment of the invention may include at least one light source and at least one detector are positioned in one ferrule adapted to be introduced into said material, or corresponding light guides conducting light to or from the sources and detectors.

Alternatively at least one light source and said at least one detector, or corresponding light guides are positioned in separate ferrules being adapted to be introduced into said material. The ferrules may then be mounted on a common unit having a predetermined distance between them, e.g. constituting a pronged configuration, or may be mounted on a common unit having an adjustable distance between them.

The analysis means may comprise a storage including a number of different characteristics, e.g. distinguishing types of meat, fish, etc, and being adapted to indicate the condition of a selected material. It may also be used to detect the type of material or properties such as fat- or water content or age of the material, and thus may be programmed to use different conditions, e.g. when barbequing a piece of meat, depending on the initial condition of the meat.

In addition the system may include temperature measuring means on the ferrule for measuring the temperature inside the material, and /or moisture measuring means for measuring the moisture of the material. This way the information about the status is increased and user control over the status of the material, e.g. meat, is improved.

The present invention thus provides a system for measuring the properties of an organic material, e.g. meat, comprising at least one light source such as LED, lasers of a wide band source, emitting light within at least two chosen wavelength ranges. As discussed above the light source unit is coupled to a light guide in a ferrule being adapted to be introduced into said material, the light sources emitting light into the material in a chosen sequence. The light within the emitted light ranges is received after having passed through a chosen length in said material, where the signal may be received by separate detectors or a spectrometer capable of separating the wavelength ranges. The received signals are analysed for monitoring the attenuation at each wavelength ranges and the relative changes between them, thus evaluating the condition of the material based on the measured attenuation due to the combination of scattering and absorption in the material in said at least two differently-evolving wavelength ranges.

The low power consumption of the LEDs, detectors, and supporting electronics makes a battery powered, self-contained and handheld unit possible, which is similar in size to a common electronic thermometer.

As the optical measurements may be performed faster than the traditional temperature measurements the unit may be used to measure the conditions at different places and/or depths in the material essentially in real time. It may also be provided with means of any known type for measuring the depth in which it is inserted into the material and thus provide a profile of the material during the insertion. Thus for example crust thickness may be measured. The measurements may also be recorded in the unit or associated equipment for future reference.

## Claims

1. Measuring system for measuring the properties of an organic material (8), e.g. meat, comprising a light source unit (4) having at least two LED sources (11) for emitting light, each one of the at least two LED sources (11) having a chosen range of wavelengths, the system also comprising a ferrule (3) and a first light guide (1), the light source unit being coupled to the first light guide (1) in the ferrule (3), the first light guide (1) in the ferrule (3) being adapted to be introduced into said material, the first light guide being connected from the light source unit to at least one point into said organic material (8),
the system also comprising detector means (2,5) adapted to receive light in at least one point within the organic material (8) and within said at least two wavelength ranges (21,22,23) comprised within said emitted range of wavelengths, having passed through a chosen length in said material,
wherein said detector means (2,5) comprises at least one light sensor (5) and a second light guide (2), the second light guide (2) extending at least along a part of the length of the ferrule (3) for guiding light from said organic material to the at least one light sensor (5), **characterised in that**
the light guide (1,2) ends are not being aimed directly at each other so that light received by the detector means (2,5) includes light that has been scattered before reaching the detector means (2,5),
the LEDs (11) being adapted to emit light in a chosen sequence thus emitting a sequence of wavelength ranges, and
the system includes analyzing means configured for measuring said received light in the wavelength ranges (21,22,23) in said sequence for evaluating the state of the material based on the relative changes in the measured attenuation due to the combination of scattering and absorption in the material between said at least two ranges of wavelengths of light scattered in the material.

2. Measuring system according to claim 1, wherein the wavelength ranges are chosen based on known chemical reactions in the material during heating, so as to provide an indication of state of condition during the heating.

3. Measuring system according to claim 2, wherein the material (8) is meat and the chemical reactions are related to the preparation of meat, e.g. indicating the colour of the prepared meat.

4. Measuring system according to claim 3, wherein the wavelength ranges (21,22,23) are in the ranges being optimized for measuring protein denaturation during heating, including near IR, especially 900-1000nm.

5. Measuring system according to claim 3, wherein the wavelength ranges (21,22,23) are in the ranges of 600-650nm, 700-720nm, 750-780nm and 790-840nm.

6. Measuring system according to claim 1, wherein the analysis performed by said analyzing means (6) is based on a cluster analysis based on the measured optical characteristics of the material.

7. Measuring system according to claim 1, wherein said chosen sequence includes at least one period with all LEDs off to compensate for surrounding light.

8. Measuring system according to claim 1, comprising one detector (12) for measuring the received light for each wavelength range.

9. Measuring system according to claim 1, comprising at least two detectors (12) or light sources (11) for each wavelength range, being positioned so as to define a number of different propagation lengths through the material between the light sources and detectors.

10. Measuring system according to claim 1, wherein said at least one light source (11) and at least one detector (12) are positioned in one ferrule adapted to be introduced into said material.

11. Measuring system according to claim 1, wherein said at least one light source (11) and said at least one detector (12) are positioned in separate ferrules being adapted to be introduced into said material.

12. Measuring system according to claim 11, wherein the ferrules (3) are mounted on a common unit having a predetermined distance between them, e.g. constituting a pronged configuration.

13. Measuring system according to claim 11, wherein the ferrules are mounted on a common unit having an adjustable distance between them.

14. Measuring system according to claim 1, wherein said analysis means (6) comprises a storage including a number of different characteristics, e.g. distinguishing types of meat, fish, etc, and being adapted to indicate the condition of a selected material.

15. Measuring system according to claim 1, also including temperature measuring means for measuring the temperature of the material.

16. Measuring system according to claim 1, also including moisture measuring means for measuring the moisture of the material.

## Patentansprüche

1. Messsystem zum Messen der Eigenschaften eines organischen Materials (8), z. B. Fleisch, umfassend eine Lichtquelleneinheit (4), die mindestens zwei LED-Quellen (11) zum Emittieren von Licht aufweist, wobei jede der mindestens zwei LED-Quellen (11) einen ausgewählten Bereich von Wellenlängen aufweist, das System ebenso umfassend eine Endhülse (3) und einen ersten Lichtleiter (1), wobei die Lichtquelleneinheit mit dem ersten Lichtleiter (1) in der Endhülse (3) gekoppelt ist, wobei der erste Lichtleiter (1) in der Endhülse (3) angepasst ist, um in das Material eingeführt zu werden, wobei der erste Lichtleiter von der Lichtquelleneinheit zu mindestens einem Punkt in das organische Material (8) verbunden ist,
das System ebenso umfassend ein Detektormittel (2,5), das angepasst ist, um Licht in mindestens einem Punkt innerhalb des organischen Materials (8) und innerhalb der mindestens zwei Wellenlängenbereiche (21,22,23), die innerhalb des emittierten Wellenlängenbereichs enthalten sind, zu empfangen, das in dem Material durch eine ausgewählte Länge hindurchgegangen ist,
wobei das Detektormittel (2,5) mindestens einen Lichtsensor (5) und einen zweiten Lichtleiter (2) umfasst, wobei sich der zweite Lichtleiter (2) mindestens entlang eines Teils der Länge der Endhülse (3) zum Leiten von Licht von dem organischen Material zu mindestens einem Lichtsensor (5) erstreckt,
**dadurch gekennzeichnet, dass**
die Enden des Lichtleiters (1,2) nicht direkt aufeinander gerichtet werden, sodass Licht, das durch das Detektormittel (2,5) empfangen wird, Licht einschließt, das vor Erreichen des Detektormittels (2,5) gestreut wurde,
die LEDs (11) angepasst sind, um Licht in einer ausgewählten Sequenz zu emittieren, womit eine Sequenz von Wellenlängenbereichen emittiert wird, und
das System ein Analysieren von Mitteln einschließt, die zum Messen des empfangenen Lichts in den Wellenlängenbereichen (21,22,23) in der Sequenz zum Auswerten des Zustands des Materials basierend auf den relativen Änderungen der gemessenen Attenuation aufgrund der Kombination von Streuung und Absorption in dem Material zwischen den mindestens zwei Wellenlängenbereichen von Licht, das in dem Material gestreut wird, konfiguriert sind.

2. Messsystem nach Anspruch 1, wobei die Wellenlängenbereiche basierend auf bekannten chemischen Reaktionen in dem Material während des Erwärmens ausgewählt werden, um eine Angabe der Beschaffenheit während des Erwärmens bereitzustellen.

3. Messsystem nach Anspruch 2, wobei das Material (8) Fleisch ist und die chemischen Reaktionen sich auf die Zubereitung von Fleisch beziehen, z. B. die Farbe des zubereiteten Fleisches angeben.

4. Messsystem nach Anspruch 3, wobei die Wellenlängenbereiche (21,22, 23) in den Bereichen, die zum Messen der Proteindenaturierung während des Erwärmens optimiert sind, einschließlich nahe IR, insbesondere 900-1000 nm liegen.

5. Messsystem nach Anspruch 3, wobei die Wellenlängenbereiche (21,22,23) in den Bereichen 600-650 nm, 700-720 nm, 750-780 nm und 790-840 nm liegen.

6. Messsystem nach Anspruch 1, wobei die durch die Analysemittel (6) durchgeführte Analyse auf einer Clusteranalyse basierend auf den gemessenen optischen Eigenschaften des Materials basiert.

7. Messsystem nach Anspruch 1, wobei die ausgewählte Sequenz mindestens einen Zeitraum mit allen LEDs aus einschließt, um Umgebungslicht zu kompensieren.

8. Messsystem nach Anspruch 1, umfassend einen Detektor (12) zum Messen des empfangenen Lichts für jeden Wellenlängenbereich.

9. Messsystem nach Anspruch 1, umfassend mindestens zwei Detektoren (12) oder Lichtquellen (11) für jeden Wellenlängenbereich, die so positioniert sind, dass sie eine Anzahl unterschiedlicher Ausbreitungslängen durch das Material zwischen den Lichtquellen und den Detektoren definieren.

10. Messsystem nach Anspruch 1, wobei die mindestens eine Lichtquelle (11) und der mindestens eine Detektor (12) in einer Endhülse positioniert sind, die angepasst ist, um in das Material eingeführt zu werden.

11. Messsystem nach Anspruch 1, wobei die mindestens eine Lichtquelle (11) und der mindestens eine Detektor (12) in separaten Endhülsen positioniert sind, die angepasst sind, um in das Material eingeführt zu werden.

12. Messsystem nach Anspruch 11, wobei die Endhülsen (3) auf einer gemeinsamen Einheit montiert sind, die einen vorbestimmten Abstand zwischen ihnen, z. B. eine zackige Konfiguration bilden, aufweisen.

13. Messsystem nach Anspruch 11, wobei die Endhülsen auf einer gemeinsamen Einheit montiert sind, die einen einstellbaren Abstand zwischen ihnen aufweist.

14. Messsystem nach Anspruch 1, wobei das Analysemittel (6) einen Speicher umfasst, einschließlich einer Anzahl unterschiedlicher Eigenschaften, z. B. Unterscheidungsarten von Fleisch, Fisch usw. und angepasst ist, um die Beschaffenheit eines ausgewählten Materials anzugeben.

15. Messsystem nach Anspruch 1, das ebenso Temperaturmessmittel zum Messen der Temperatur des Materials einschließt.

16. Messsystem nach Anspruch 1, das ebenso Feuchtigkeitsmessmittel zum Messen der Feuchtigkeit des Materials einschließt.

## Revendications

1. Système de mesure destiné à mesurer les propriétés d'une matière organique (8), par ex. de la viande, comprenant une unité de sources de lumière (4) ayant au moins deux sources de DEL (11) pour émettre de la lumière, chacune des au moins deux sources de DEL (11) ayant une gamme de longueurs d'onde choisie, le système comprenant également une ferrule (3) et un premier guide de lumière (1), l'unité de source de lumière étant couplée au premier guide de lumière (1) dans la ferrule (3), le premier guide de lumière (1) dans la ferrule (3) étant adapté pour être introduit dans ladite matière, le premier guide de lumière étant relié depuis l'unité de source de lumière à au moins un point dans ladite matière organique (8),
le système comprenant également un moyen détecteur (2, 5) adapté pour recevoir de la lumière en au moins un point au sein de ladite matière organique (8) et dans lesdites au moins deux gammes de longueurs d'onde (21, 22, 23) comprises dans ladite gamme de longueurs d'onde émise, ayant parcouru une longueur choisie dans ladite matière,
dans lequel ledit moyen détecteur (2, 5) comprend au moins un capteur de lumière (5) et un second guide de lumière (2), le second guide de lumière (2) s'étendant au moins sur une partie de la longueur de la ferrule (3) pour guider la lumière depuis ladite matière organique vers au moins un capteur de lumière (5),
**caractérisé en ce que**
les extrémités du guide de lumière (1, 2) ne sont pas dirigées directement l'une vers l'autre de sorte que la lumière reçue par le moyen détecteur (2, 5) comporte une lumière qui a été diffusée avant d'atteindre le moyen détecteur (2, 5),
les DEL (11) étant adaptées pour émettre une lumière dans une séquence choisie, émettant ainsi une séquence de gammes de longueurs d'onde, et
le système comporte des moyens d'analyse configurés pour mesurer ladite lumière reçue dans les gammes de longueurs d'onde (21, 22, 23) dans ladite séquence pour évaluer l'état de ladite matière sur la base des changements relatifs dans l'atténuation mesurée dus à la combinaison d'une diffusion et d'une absorption dans la matière entre lesdites au moins deux gammes de longueurs d'onde de lumière diffusée dans la matière.

2. Système de mesure selon la revendication 1, dans lequel les gammes de longueurs d'onde sont choisies sur la base de réactions chimiques connues dans la matière pendant le chauffage, de manière à fournir une indication d'état pendant le chauffage.

3. Système de mesure selon la revendication 2, dans lequel la matière (8) est de la viande et les réactions chimiques sont liées à la préparation de la viande, par ex. indiquant la couleur de la viande préparée.

4. Système de mesure selon la revendication 3, dans lequel les gammes de longueurs d'onde (21, 22, 23) sont dans les gammes optimisées pour mesurer une dénaturation des protéines pendant le chauffage, y compris le proche IR, en particulier de 900 à 1000 nm.

5. Système de mesure selon la revendication 3, dans lequel les gammes de longueurs d'onde (21, 22, 23) sont comprises dans les plages de 600 à 650 nm, 700 à 720 nm, 750 à 780 nm et 790 à 840 nm.

6. Système de mesure selon la revendication 1, dans lequel l'analyse effectuée par ledit moyen d'analyse (6) est basée sur une analyse par groupe basée sur les caractéristiques optiques mesurées de la matière.

7. Système de mesure selon la revendication 1, dans lequel ladite séquence choisie comporte au moins une période avec toutes les DEL éteintes pour compenser une lumière environnante.

8. Système de mesure selon la revendication 1, comprenant un détecteur (12) pour mesurer la lumière reçue pour chaque gamme de longueurs d'onde.

9. Système de mesure selon la revendication 1, comprenant au moins deux détecteurs (12) ou sources de lumière (11) pour chaque gamme de longueurs d'onde, positionnés de manière à définir un certain nombre de longueurs de propagation différentes à travers la matière entre les sources de lumière et les détecteurs.

10. Système de mesure selon la revendication 1, dans lequel ladite au moins une source de lumière (11) et ledit au moins un détecteur (12) sont positionnés dans une ferrule adaptée pour être introduite dans ladite matière.

11. Système de mesure selon la revendication 1, dans lequel ladite au moins une source de lumière (11) et ledit au moins un détecteur (12) sont positionnés dans des ferrules distinctes adaptées pour être introduites dans ladite matière.

12. Système de mesure selon la revendication 11, dans lequel les ferrules (3) sont montées sur une unité commune en ayant une distance prédéterminée entre elles, par ex. constituant une configuration à branches.

13. Système de mesure selon la revendication 11, dans lequel les ferrules sont montées sur une unité commune en ayant une distance réglable entre elles.

14. Système de mesure selon la revendication 1, dans lequel ledit moyen d'analyse (6) comprend une mémoire comportant un certain nombre de caractéristiques différentes, par ex. distinguant des types de viande, de poisson, etc., et adaptée pour indiquer l'état d'une matière sélectionnée.

15. Système de mesure selon la revendication 1, comportant également des moyens de mesure de température pour mesurer la température de ladite matière.

16. Système de mesure selon la revendication 1, comportant également des moyens de mesure d'humidité pour mesurer l'humidité de ladite matière.
